# EUROPÄISCHE PATENTSCHRIFT

(11) **EP 3 338 814 B1**
(45) Veröffentlichungstag und Bekanntmachung des Hinweises auf die Patenterteilung: **03.03.2021**
(21) Anmeldenummer: 17208765.2
(22) Anmeldetag: 20.12.2017
(51) Int. Cl.: A61L 27/04, A61L 27/06, A61L 27/54, A61L 31/02, A61L 31/16, A61L 27/56, A61L 31/14, B29C 64/153, B33Y 70/00

(54) **STRUKTURIERTES LEICHTMETALLBAUTEIL**
STRUCTURED LIGHTWEIGHT METAL COMPONENT
COMPOSANT EN MÉTAL LÉGER STRUCTURÉ

(30) Priorität: 22.12.2016 DE 102016226048
(43) Veröffentlichungstag der Anmeldung: 27.06.2018
(73) Patentinhaber: Meotec GmbH, 52068 Aachen (DE)
(72) Erfinder: Kopp, Alexander, 52064 Aachen (DE); Müther, Max, 52070 Aachen (DE)
(74) Vertreter: FARAGO Patentanwälte

(56) Entgegenhaltungen:
- WO-A1-2015/144702
- ERIC ALEXANDER LEWALLEN ET AL: "Biological Strategies for Improved Osseointegration and Osteoinduction of Porous Metal Orthopedic Implants", TISSUE ENGINEERING PART B-REVIEWS, Bd. 21, Nr. 2, 1. April 2015 (2015-04-01), Seiten 218-230, XP055453664, US ISSN: 1937-3368, DOI: 10.1089/ten.teb.2014.0333

## Beschreibung

### Gebiet der Erfindung

Die Erfindung bezieht sich auf ein strukturiertes Leichtmetallbauteil und auf ein Verfahren zur Herstellung dieses Leichtmetallbauteils.

### Hintergrund der Erfindung

Die gezielte schichtweise Herstellung von Bauteilen aus Lösungen bei der schnellen Produktentwicklung (sogenanntes "Rapid Prototyping" oder "Rapid Manufacturing") wird mittlerweile in den unterschiedlichsten Technologisbereichen angewendet. Gerade komplizierte Bauteile, die in geringer Stückzahl oder gar als Unikat benötigt werden, können mit solchen Verfahren unkompliziert und sehr variabel hergestellt werden. Eine bekannte Fertigungstechnik dafür ist Stereolithographie, mit der beispielsweise Motorgetriebe, Architekturmodelle, Gerätegehäuse oder gar Implantate für den menschlichen Körper in der jeweils gewünschten Form hergestellt werden können. Das Bauteil wird dabei am Computer entworfen und die entsprechenden CAD-Daten zur Steuerung der schichtweise Belichtung des aus der Suspension herausgezogenen Bauteils verwendet, um das Bauteil den Computerentwurf nachzubauen.

Auf dem Gebiet der Implantate für den menschlichen Körper beispielsweise zum Ersetzen fehlender Knochen oder zur Stabilisierung von Knochenfrakturen benötigen Implantate eine hohe mechanische Stabilität und eine biologische Kompatibilität. Neben diesen Eigenschaften muss auch der Komfort der Patienten berücksichtigt werden. Häufig klagen Patienten über Unbehagen, weil sich Implantate unter Sonneneinstrahlung erwärmen oder die Unterschiede der mechanischen Eigenschaften vom Implantat und Knochen zu Problemen führen, was insbesondere bei derzeit üblichen metallischen Implantaten auftritt. Mittels Stereolithographie können dagegen auch oxydische Keramiken wir ZrO₂ oder Al₂O₃ für die Verwendung als Implantate hergestellt werden. Keramiken aus ZrO₂ oder Al₂O₃ besitzen zwar eine gute mechanische Stabilität bei statischer Belastung, sind aber spröde und empfindlich gegen Stoßbelastungen.

Implantate aus gewöhnlichen Materialien verbleiben im Körper, da dieser sie nicht abbauen kann. Daher werden gerne resorbierbare Materialien wie resorbierbare Polymere, beispielsweise Polylactide (PLA) und deren CoPolymere für Implantate verwendet, weil sich diese im Körper auflösen und somit keine eventuellen Folgeoperationen zum Entfernen der Implantate notwendig werden. Allerdings begrenzen die mechanischen Eigenschaften der resorbierbaren Polymere deren Einsatzgebiet bei den Implantaten auf Platten, Schrauben oder Nahtmaterialien.

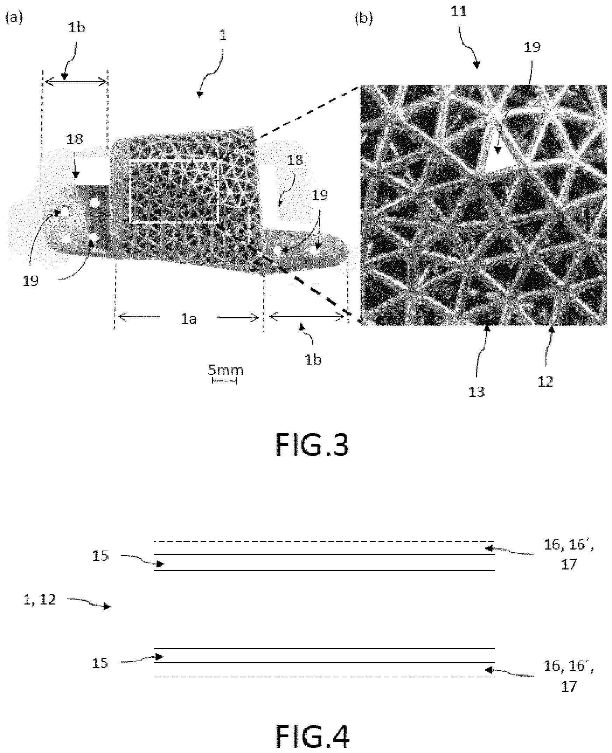

Die WO 2015/144702 A1 offenbart ein Verfahren zur Herstellung eines porösen Titanbauteils, bei dem Titanpulver mit NaCI-Partikeln und einem Bindematerial gemischt wird, die Mischung bei 200-400 MPa kompaktiert wird, das Bindematerial und das Salz durch thermische Behandlung und Spülen mit destilliertem Wasser entfernt werden und das erhaltene Material bei 1200-1400°C gesintert wird. Die Struktur ist hier lediglich durch die innere Porosität des Metallteils gegeben, die durch die Zugabe des NaCI-Salzes zur Metallpulver-/Bindermischung mit anschließenden Auswaschen und Sintern resultiert (siehe Figur 4). Es handelt sich also lediglich um einen porösen Körper. Eine Struktur mit Streben, die über Kreuzungs- bzw. Verbindungspunkte verbunden sind ist und dadurch Leerräume zwischen den Streben bilden wird in der WO'702 nicht offenbart.

Lewallen et al, (Tissue Engineering: Part B, 21 (2) Seiten 218-230, 2015) offenbart poröse Implantate auf Basis von Titan oder Magnesium, wobei sie lehrt, dass diese porösen Biomaterialien durch unterschiedliche Technologien wie z.B. Stereolithographie gefertigt werden können. Ein Backprozess, der zum Aufbrechen einer Oxidschicht des Pulvermaterials führt, wird von Levallen nicht offenbart.

Es wäre daher wünschenswert, ein Bauteil aus einem Material zur Verfügung zu haben, das als Implantat verwendbar ist, ohne dabei die voranstehenden Nachteile aufzuweisen.

### Zusammenfassung der Erfindung

Es ist daher eine Aufgabe der vorliegenden Erfindung, ein Bauteil aus einem Material zur Verfügung zu stellen, das mechanisch sowohl statisch als auch dynamisch stabil und belastbar ist und geeignet ist, im Falle eines Implantats dem Patienten einen besseren Tragekomfort zu bieten.

Diese Aufgabe wird gelöst durch ein strukturiertes Leichtmetallbauteil aus einem Material vorzugsweise geeignet zum Einsatz als Implantat, das aus einem Pulvermaterial umfassend Magnesium und/oder Titan mittels Stereolithographie zu einem vorstrukturierten Vorformling und mittels eines geeigneten Backprozesses oberhalb einer Temperatur geeignet zum Aufbrechen einer Oxydschicht des Leichtmetallmaterials zu dem Leichtmetallbauteil gefertigt ist und das Material des fertigen strukturierten Leichtmetallbauteils zumindest zum überwiegenden Teil aus Magnesium und/oder Titan besteht, wobei das strukturierte Leichtmetallbauteil eine netzartige Struktur aus Streben aus dem Material des Leichtmetallbauteils und dazwischen vorhandene Leerräume umfasst, vorzugsweise ist die netzartige Struktur als Volumennetz ausgebildet, und wobei die netzartige Struktur (11) mehrere Kreuzungs- und/oder Verbindungspunkte (14) der Streben miteinander aufweist.

Erfindungsgemäß gebackene Bauteile aus Titan und Magnesium weisen gute Eigenschaften hinsichtlich Festigkeit und Biokompatibilität auf. Durch das Backen besitzt das erfindungsgemäße Leichtbauteil eine hohe Festigkeit. Geeignete Temperaturen zum Aufbrechen einer Oxidschicht des Leichtmetallmaterials liegen im Fall von Titan bei oberhalb 400°C, Temperaturen oberhalb von 650°C brechen die Oxidschicht bei Magnesium auf. Diese Temperaturen sind geeignete Temperaturen im Sinne der Erfindung. Hierbei wird die geeignete Temperatur auf ein Temperaturintervall nach oben so begrenzt, dass die Leichtmetallpulver nicht oder zumindest nicht vollständig aufgeschmolzen werden. Bei Titan oder Titanoxid als Pulvermaterial ist eine Backtemperatur bis knapp unterhalb der jeweiligen Schmelztemperatur von 1668°C beziehungsweise 1843°C möglich, wobei zur vereinfachten Prozessführung bei Titanmaterialien vorteilhafterweise Backtemperaturen zwischen 1000°C und 1400°C verwendet werden. Magnesium besitzt dagegen eine Schmelztemperatur von 650°C. Daher kann der Teil des Magnesiumpulvers, der bereits reduziert wurde, während des Backvorgangs zumindest zum Teil angeschmolzen werden, daher sollte hier die Backtemperatur nicht zu hoch oberhalb 650°C gewählt werden. Sofern Magnesiumoxid mit einer Schmelztemperatur von 2852°C als Pulvermaterial verwendet wird, kann zumindest am Anfang des Backvorgangs eine deutlich höhere Backtemperatur verwendet werden als bei nicht-oxidiertem Magnesiumpulver. Im Gegensatz zu oxydischen Keramiken, die typischerweise sehr spröde sind, sind die erfindungsgemäßen Leichtmetallbauteile aus Titan oder Magnesium sehr stabil, sowohl unter statischer als auch unter dynamischen Belastung und hervorragend bio-kompatibel. Titan ist zudem sehr korrosionsbeständig. Durch die Strukturierung erwärmen sich die erfindungsgemäßen Leichtmetallbauteile weniger schnell bei Sonneneinstrahlung im Gegensatz zu Implantaten aus Vollmaterial, was im Falle von Implantaten den Tragekomfort zusätzlich zum geringen Gewicht durch die Struktur des Leichtmetallbauteils steigert. Die erfindungsgemäßen Leichtmetallbauteile können unter anderem als Leichtbauteile in der Automobilindustrie sowie in der Luft- und Raumfahrttechnik verwendet werden, aber ebenso auch durch die leichte Erzeugbarkeit komplexer Strukturen für robuste Gehäusestrukturen aller Art und in der Ersatzteilproduktion.

Im Gegensatz zu Titan ist Magnesium sehr gut im menschlichen Körper resorbierbar, da es als Mineral in fast allen Körperzellen vorhanden ist und Magnesium oder Legierungen aus Magnesium deutlich fester sind als beispielsweise resorbierbare Polymere. Magnesium-Implantate können somit vom Körper absorbiert und von natürlichen Knochen ausgefüllt werden. Somit verbleiben Implantate aus Magnesium nicht mehr als Fremdkörper im menschlichen Körper und vermeiden so eventuelle spätere Komplikationen. Ein Material wird dabei als resorbierbar bezeichnet, wenn sich das Material in einer Körperumgebung im jeweiligen Organismus auflöst und das Material von Organismus zumindest zum Teil aufgenommen wird. Bauteile aus Titan oder Magnesium sind somit hervorragend zum Einsatz als Implantat geeignet.

Erfindungsgemäß umfasst das Leichtmetallbauteil eine netzartige Struktur aus Streben aus dem Material des Leichtmetallbauteils und dazwischen vorhandene Leerräume. Die netzartige Struktur kann dabei zwei- oder dreidimensional ausgeführt sein. In einer Ausführungsform ist die netzartige Struktur dabei als Volumennetz ausgebildet, das somit eine dreidimensionale Gitterstruktur darstellt. Die netzartige Struktur ermöglicht eine große Stabilität der Struktur bei gleichzeitig geringem Gewicht für alle Anwendungen des Leichtmetallbauteils. Insbesondere für die Anwendung als Implantat regt die netzartige Struktur das Knochenwachstum an. Hierbei ist die Möglichkeit des Einwachsens des Knochens bei gleichzeitigem Stützen des Defekts durch das Implantat gegeben. Hierbei werden die Begriffe zweidimensional und dreidimensional nicht im strengen mathematischen Sinn verwendet. Als zweidimensionale Struktur ist dessen flächige Ausdehnung um mehr als eine Größenordnung (um einen Faktor 10), vorzugsweise um mehr als zwei Größenordnungen (um einen Faktor 100), größer als die Ausdehnung senkrecht zur flächigen Ausdehnung. Bei einer dreidimensionalen Struktur variiert die Ausdehnung der netzartigen Struktur in alle Raumrichtungen nicht um mehr als eine Größenordnung. Ein Volumennetz bezeichnet dabei eine dreidimensionale Struktur, die nicht nur eine netzartige Struktur entlang einer das Leichtmetallbauteil einhüllenden Oberfläche besitzt, sondern die sich auch durch das Volumen hindurch fortsetzt.

In einer Ausführungsform besitzen die Streben einen Durchmesser von 100µm bis 1500µm und/oder die Leerräume einen Durchmesser zwischen 200µm und 1500µm besitzen. Mit diesen Parametern sind Leichtmetallbauteile aus dieser netzartigen Struktur mit einer Strukturdichte von 10%-50% im Vergleich zu einem kompakten Körper herstellbar, was für alle Anwendungen, bei denen das Gewicht eine kritische Größe darstellt, wichtig ist, wie beispielsweise in der Automobilindustrie, in der Luft- und Raumfahrttechnik und bei Implantaten. Der Begriff "Durchmesser" bezeichnet hier nicht, dass die Streben oder die Leerräume eine kreis- oder kugelartige Form beziehungsweise Querschnittsfläche besitzen müssen. Der Durchmesser bezeichnet hier den durchschnittlichen Abstand gegenüberliegender Punkte in der jeweiligen Strebe oder dem jeweiligen Leerraum gemittelt aus allen Abständen der jeweils gegenüberliegenden Punkte relativ zum geometrischen Zentrum der jeweiligen Form.

Erfindungsgemäß weist die netzartige Struktur mehrere Kreuzungs- und/oder Verbindungspunkte der Streben miteinander auf. Das erhöht die Stabilität insbesondere bei Volumennetzen, da hier die Kreuzungs- und Verbindungspunkte nicht nur an der Oberfläche des daraus definierten Leichtmetallbauteils (was alleine schon die Stabilität erhöht), sondern auch innerhalb des Volumens des Leichtmetallbauteils liegen, sodass sich die Streben gegeneinander abstützen und somit einer besonders hohen Stabilität des Volumennetzes bereitstellen. In einer weiteren Ausführungsform sind die Streben und Leerräume des Volumennetzes so angeordnet, dass die netzartige Struktur mindestens einen Lichtkanal aufweist, durch den externes Licht das Leichtmetallbauteil passieren kann. Dieser Lichtkanal kann eine beliebige Richtung durch das Leichtmetallbauteil aufweisen. Das Leichtmetallbauteil ist in dieser Ausführungsform nicht opak, also lichtdurchlässig. Die netzartige Struktur kann weitere Kanäle gebildet durch die entsprechend ausgerichtete Anordnung der Streben und Leerräume gegebenenfalls für andere Zwecke aufweisen.

In einer weiteren Ausführungsform umfasst das Leichtmetallbauteil mindestens einen ersten Bereich und einen zweiten Bereich, wobei die netzartige Struktur im ersten Bereich und eine kompakte Struktur zumindest im zweiten Bereich angeordnet sind. Das Leichtmetallbauteil kann in anderen Ausführungsformen auch weitere dritte, vierte oder weitere Bereiche umfassen, in denen je nach Anwendung netzartige Strukturen oder kompakte Strukturen angeordnet sind. Die verschiedenen Bereiche des Leichtmetallbauteils sind dabei durch den Herstellungsprozess fest miteinander verbunden. Die kompakten Strukturen können beispielsweise der Befestigung des Leichtmetallbauteils auf einer dafür vorgesehenen Unterlage dienen. Dafür können die kompakten Strukturen beispielsweise Bohrungen oder Löcher, gegebenenfalls Gewindelöcher, in geeigneter Anzahl und Anordnung aufweisen. Im Falle eines Implantats kann die Unterlage Knochenmaterial sein.

In einer weiteren Ausführungsform weist das Material des Leichtmetallbauteils eine Oberflächenoxidschicht auf. Diese Oberflächenoxidschicht passiviert das Leichtmetallbauteil gegenüber Umwelteinflüssen von außen auf das Leichtmetallbauteil. Die Oxidschicht kann dabei eine Dicke von 2µm bis 500µm aufweisen. Die Oxidschicht kann dabei künstlich erzeugt oder natürlich gewachsen sein. Eine künstliche Oxidschicht kann beispielsweise mittels Anodisation oder plasmaelektrolytische Oxidation (PEO) erzeugt werden. Die plasmaelektrolytische Oxidation (PEO) wird unter anderem auch als plasmachemische Oxidation (PCO), Anodisation unter Funkenbildung (ANOF) oder als sogenannte Micro-Arc-Oxidation (MAO) bezeichnet. Die natürlich gewachsene Oxidschicht kann beispielsweise an Luft oder in einem Gas mit kontrolliertem Sauerstoffanteil aufwachsen. Die bevorzugte Dicke dieser Oxidschicht beträgt 5 - 20µm. Beispielsweise kann die Oxidschicht eine mittlere Dicke von 10µm besitzen. Im Falle von Magnesium als Material des Leichtmetallbauteils kann die Oxidschicht ein Auflösen des Leichtmetallbauteils in wässriger Umgebung verhindern oder den Auflösungsprozess zeitlich kontrollieren, was je nach Anwendung entscheidend sein kann, beispielsweise bei Leichtmetallbauteilen als Implantate.

In einer Ausführungsform ist das Leichtmetallbauteil ein Implantat. Titan und Magnesium weisen gute Eigenschaften hinsichtlich Festigkeit und Biokompatibilität auf. Titan ist zudem sehr korrosionsbeständig. Die Struktur des Leichtbauteils verbessert zudem den Tragekomfort bei Patienten. Durch die Struktur erwärmen sich die erfindungsgemäßen Leichtmetallbauteile weniger schnell bei Sonneneinstrahlung, was den Tragekomfort zusätzlich steigert. Im Gegensatz zu Titan ist Magnesium zudem sehr gut im menschlichen Körper resorbierbar, da es als Mineral in fast allen Körperzellen vorhanden ist und Magnesium oder Legierungen aus Magnesium deutlich fester sind als beispielsweise resorbierbare Polymere. Magnesium-Implantate können somit vom Körper absorbiert und in natürlichen Knochen umgewandelt oder ersetzt werden. Somit verbleiben Implantate aus Magnesium nicht mehr als Fremdkörper im menschlichen Körper und vermeiden so eventuelle spätere Komplikationen.

In einer Ausführungsform weist das Leichtmetallbauteil als Implantat zusätzlich eine Beschichtung mit antibakterieller Wirkung oder ein Antibiotikum auf oder umfasst einen Anteil von bis zu 10% Silber. Um eine antibakterielle Wirkung zu erzeugen kann auch die entsprechende Beschichtung einen Silberanteil von bis zu 10% umfassen. Bei der Applikation als Implantat erfolgt durch solche eine antibakterielle Schicht eine kontrollierte Freisetzung der biofunktionalen Substanzen, was den Heilungsprozess fördert. Das Leichtmetallbauteil kann in einer weiteren Ausführungsform statt der voranstehenden Beschichtung oder als zusätzliche Beschichtung eine Beschichtung aus einem Material der Gruppe nativer oder künstlicher Kollagene, Fibrine, natürlicher oder künstlicher Seiden, natürlicher Proteine oder Polymere wie PLA, PDLA, PLLA, PTFE oder einer Sol-Gel-Beschichtung, aufweisen. Diese Schicht kann das Leichtmetallbauteil schützen oder seine Oberflächeneigenschaften anpassen. Die Dicken der voranstehend aufgeführten Beschichtungen können zwischen 0,1µm und 10µm liegen.

In einer Ausführungsform besteht das Leichtmetallbauteil aus Magnesium oder einer Magnesiumlegierung, die bis zu 1,5% Zink, bis zu 1,5% Calcium, bis zu 5% Yttrium und/oder bis zu 4% seltene Erden umfasst. Die voranstehenden Prozentangaben beziehen sich auf Gewichtsprozente (Gew%). Beispielweise können Magnesiumlegierungen mit einem Anteil von 4% Yttrium und 3% Seltene Erden ohne Zink und Kalzium (sogenanntes WE43) oder mit einem Anteil von 1% Zink und 1% Kalzium ohne Yttrium und Seltene Erden (sogenanntes ZX11) verwendet werden. Der Begriff "ohne" bezeichnet eine Materiallegierung, die die mit "ohne" bezeichneten Materialien nicht oder höchstens als Verunreinigungen kleiner 0,1% umfasst. Die im Vergleich zu Titan geringere Festigkeit von Magnesium ist beispielsweise für Implantate erwünscht, da die mechanische Festigkeit und das Elastizitätsmodul von Magnesium und der meisten seiner Legierungen vergleichbar mit dem menschlichen Knochen ist. Magnesium als körpereigener Bestandteil erfüllt demnach aufgrund deiner uneingeschränkten Biokompatibilität und knochenähnlichen Festigkeit alle Voraussetzungen für einen Implantatwerkstoff. Die Zusätze in einer Magnesiumlegierung beeinflussen die Korrosionsbeständigkeit von Magnesium, dessen Festigkeit und weitere mechanische Eigenschaften. Durch die geeignete Einstellung des Anteils an Zink, Calcium, Yttrium und/oder seltener Erden in den voranstehend angeführten Anteilsintervallen lassen sich die Eigenschaften der Magnesiumlegierung auf die jeweilige Anwendung hin optimieren, bei Implantaten beispielsweise je nach Art der Funktion des Knochens, der durch das Implantat ersetzt oder ergänzt werden soll. In einer weiteren Ausführungsform weist das Material durch den Backprozess eine Materialdichte von mehr als 90%, vorzugsweise mehr als 97%, besonders bevorzugt mehr als 99%, der jeweiligen nominalen Dichte von Magnesium oder Titan auf, je nachdem, welches Material (Magnesium oder Titan) das Leichtmetallbauteil überwiegend umfasst. Die nominale Dichte von Magnesium und Titan bezeichnet die Dichte der jeweiligen Materialien im kristallinen Festkörper bei Raumtemperatur. Das den Backprozess und die angewendete geeignete Temperatur lässt sich die Porosität im Leichtmetallbauteil auf unter 3% reduzieren.

In einer Ausführungsform ist das Leichtmetallbauteil daher ein Implantat, vorzugsweise für einen Knochenersatz. Das Implantat kann dabei als Knochenplatte, Knochenschraube, Nahtanker in Form eines kompakten Körpers oder als Netz (ein sogenanntes Mesh) oder dergleichen verwendet werden.

In einer alternativen Ausführungsform besteht das Material aus Titan oder einer Titanlegierung, wobei der Backprozess ein Sinterprozess ist und die geeignete Temperatur zwischen 400 Grad und der Schmelztemperatur von Titan liegt. Ein Sinterprozess bezeichnet dabei einen Prozess, bei dem ein Vorformling (beim Sintern auch oft auch als Grünkörper oder Grünling bezeichnet) erhitzt wird, wobei die Temperaturen jedoch unterhalb der Schmelztemperatur von Titan (1668°C) bleiben, so dass die grundsätzliche Gestalt (Form) des Vorformlings (hier Grünkörper) erhalten bleibt. Dabei kommt es aber in der Regel zu einer Schwindung, weil sich die Partikel des Titanpulvers verdichten und Porenräume aufgefüllt werden, während das organische Bindermaterial vollständig oder zumindest teilweise ausgetrieben wird. Das vorher miteinander verklebte Pulvermaterial wird durch die Erwärmung miteinander verbunden und verdichtet. Im Gegensatz zur reinen Schmelze werden hierbei jedoch die Körner des Pulvermaterials nicht aufgeschmolzen. Die Festigkeit des gesinterten Vorformlings (hier Grünkörper) wird unter anderem durch ausgebildete Hälse zwischen den Pulverpartikeln erreicht, die sich mittels Oberflächendiffusion zwischen den Pulverpartikeln bilden. Das Pulvermaterial im Vorformling (hier Grünkörper) wird durch das Sintern somit zusammengebacken, wodurch das Sintern ein Urformverfahren darstellt. Implantate aus Titan sind zwar nicht resorbierbar, aber sehr robust und derzeitiger Standard. Das Sinterverfahren ermöglicht einen schnellen und günstigen Aufbau auch sehr komplexer Strukturen. Temperaturen oberhalb 400 Grad brechen die Oxydschicht bei Titan auf.

Die Erfindung betrifft des Weiteren ein Verfahren zur Herstellung eines erfindungsgemäßen strukturierten Leichtmetallbauteils aus einem Material geeignet zum Einsatz als Implantat umfassend die Schritte
- Bereitstellen einer Suspension umfassend zumindest ein organisches Bindemittel sowie ein Pulvermaterial umfassend Magnesium und/oder Titan in einer geeigneten Konzentration und Pulvergröße, vorzugsweise mit einem Durchmesser kleiner 200µm (beispielsweise größer als 100µm oder 1µm bis 100pm, insbesondere 20µm-63µm);
- schichtweises Herstellen eines vorstrukturierten Vorformlings aus der Suspension mittels Stereolithographie mit einer Energiedichte, die geeignet ist, dass das Bindemittel das Pulvermaterial miteinander zu einer stabilen Vorstruktur verbindet; und
- Backen des Vorformlings bei einer geeigneten Temperatur geeignet zum Aufbrechen einer Oxydschicht des Pulvermaterials und zur Verbindung des Leichtmetallmaterials der Vorstruktur unter zumindest teilweisem Austrieb des Bindemittels zum fertigen strukturierten Leichtmetallbauteil, das nach erfolgtem Backen zumindest zum überwiegenden Teil aus Magnesium und/oder Titan besteht.

Das Verfahren liefert Leichtmetallbauteile, die mechanisch sowohl statisch als auch dynamisch sehr stabil und belastbar sind. Dieses Verfahren ermöglicht zudem einen schnellen und günstigen Aufbau von Leichtmetallbauteilen mit komplexen Strukturen, die in der Automobilindustrie sowie in der Luft- und Raumfahrttechnik verwendet werden können oder durch die leichte Erzeugbarkeit komplexer Strukturen für robuste Gehäusestrukturen aller Art und in der Ersatzteilproduktion anwendbar sind. Diese Leichtmetallbauteile sind zudem durch ihre Materialwahl Titan und/oder Magnesium als Implantate sehr gut geeignet.

Bei der Stereolithographie wird die obige Suspension schichtweise belichtet, sodass die belichtete Schicht aus Bindemittel und Pulverpartikeln aushärtet. Dafür ist die Energiedichte des für die Belichtung verwendeten Lichts ausreichend gewählt. Der Fachmann ist für die Einstellung der benötigten Energiedichte in der Lage, die dafür benötigten Lichtquellen, deren Betriebsparameter und Optiken geeignet zu wählen. Die ausgehärtete Schicht wird mittels einer Ziehvorrichtung über die Oberfläche der Suspension gehoben oder mittels einer Hubeinrichtung vollständig in diese eingetaucht und nachfolgend die nächste Schicht darunter oder darüber in der Suspension belichtet und ausgehärtet, wobei sich die einzelnen Schichten miteinander ebenfalls verbinden. Die für den Stereolithographie-Prozess eingesetzten organischen Bindemittel müssen unter einer geeigneten Belichtung aushärten, während sie unbelichtet flüssig bleiben. Geeignete Bindemittel für den Stereolithographie-Prozess sind beispielsweise Acrylat (beispielsweise polyfunktionale Acrylate), Acrylamide (AA), Triethanolamine (TEA), Yellowish Eosin (YE), Dimethylacrylamide (DMMA) oder Dimethacrylat oder Mischungen daraus in einer Polyvinyl- Alkohol-Lösung (PVA), vorzugsweise einer 6% PVA-Lösung, oder einer Polypropylen-Glykol-Lösung. Ein bioaktives Glaspulver kann von der Schott AG (Mainz, Germany) unter dem Namen "Vitryxx" bezogen werden. Eine geringe Viskosität der zu belichtenden Schicht kann erreicht werden, indem ein Pulver mit geringer Partikelgröße und - Verteilung verwendet wird. Ein entsprechender Glasschlicker (Glass-Slurry) mit einem Anteil an 47.7 Vol% der Glaspartikel zeigt gute Druckeigenschaften. Eine geeignete Zusammensetzung des Glasschlickers umfasst dabei beispielsweise 10 Gew% polyfunktionale Acrylate mit einer Dichte von 1,05 g/cm³, 8,2 Gew% Dimethacrylat mit einer Dichte von 1,11 g/cm³, 10,745 Gew% Polypropylen-Glykol mit einer Dichte von 1,01 g/cm³, 1 Gew% Dispergiermittel mit einer Dichte von 1,16 g/cm³, 0,005 Gew% eines lichtabsorbierenden Materials, 0,05 Gew% von Initiatormolekülen, 70 Gew% an Glaspulver mit einer Dichte von 2,7 g/cm³. Die Pulververteilung wird erreicht durch ein adäquates Dispergiermittel, beispielsweise Disperbyk-111 der Firma, BYK Additives & Instruments, Wesel, Germany. Um eine übermäßige Polymerisation durch Streulicht zu vermeiden kann ein organischer Farbstoff hinzugesetzt werden, beispielsweise Disperse Orange 3 der Firma Sigma-Aldrich, St. Louis, MO, USA. Eine homogene Schicht kann mittels eines Rührmischgerätes, beispielsweise mittels des Speedmixers DAC150.1 der Firma Hauschild, Hamm, Germany, erreicht werden.

In einem anderen Beispiel können auch Schlicker auf der Basis von radikalisch polymerisierbarem Bindemittel, Polymerisationsinitiator und Füllstoff verwendet werden, die (A) 5-65 Gew%, vorzugsweise 9 bis 65 Gew%, besonders bevorzugt 10 bis 40 Gew% polyreaktives Bindemittel, (B) 0,001 - 1,0 Gew%, vorzugsweise 0,01 bis 1,0 Gew%, besonders bevorzugt 0,1 bis 1,0 Gew% Photoinitiator und (C) 35 - 90 Gew%, vorzugsweise 50 bis 90 Gew%, besonders bevorzugt 60 bis 90 Gew% oberflächenmodifizierte Keramik- und/oder Glaskeramikpartikel enthalten.

In einem anderen Beispiel könnte die lichthärtbare Zusammensetzung für die Herstellung dreidimensionaler Körper durch Stereolithographie, 40 bis 70 Vol.-% Keramik- oder Metallpartikel, 10 bis 35 Gew% Monomer, 1 bis 10 Gew% Photoinitiator, 1 bis 10 Gew% Dispergiermittel und vorzugsweise auch Lösungsmittel, Weichmacher und Kopplungsmittel enthalten.

Als polyreaktives Bindemittel lassen sich besonders Polymerisations- und Polyadditionsharze verwenden, die in der Regel aus einer Mischung von niedermolekularen oder oligomeren Monomeren bestehen, die eine oder mehrere polyreaktionsfähige Gruppen enthalten. Im Falle von Polymerisationsharzen sind radikalisch und kationisch polymerisierbare Harze sowie Monomere für die ringöffnende Metathesepolymerisation bevorzugt. Als radikalische Polymerisationsharze lassen sich insbesondere mono- oder multifunktionelle (Meth)acrylate oder deren Mischung einsetzen. Bevorzugte Monomere sind Acrylate wie Hydroxyethyl-, Hydroxypropyl-, Benzyl-, Tetrahydrofurfuryl- oder Isobornylacrylat, sowie Di-, Tri- oder Tetraethylenglycoldiacrylat, Acrylatgruppen-terminierte Poly(ethylenglycol)e und Poly(propylenglycol)e, Hexandioldiacrylat, Trimethylolpropantriacrylat und Pentaerythrittetraacrylat. Dabei werden die di- oder multifunktionellen Acrylate in der organischen Schlickerphase vorzugsweise in Mischung mit Monoacrylaten eingesetzt. Harze für die stereolithographische Herstellung von gesinterten Keramik- oder Metallteilen können eine Viskosität von weniger als 3000 mPas besitzen. Weiterhin bevorzugte Monomere sind Acrylamide wie N-Ethylacrylamid, N,N-Dimethylacrylamid, N-(2-Hydroxyethyl)acrylamid, N,N'-Diethyl-1,3-bis(acrylamido)-propan und 1,4-Bis(acryl-amido)-butan. Dabei werden die Bisacrylamide im Vergleich zu den Monoacrylamiden im organischen Bindemittel vorzugsweise im Überschuss eingesetzt. Der Anteil an Monoacrylamiden in der Komponente (A) beträgt bevorzugt 30 Gew% oder weniger. Als kationische Polymerisationsharze lassen sich kationisch ringöffnend polymerisierbare Monomere wie z.B. Glycidylether oder cycloaliphatische Epoxide, cyclische Ketenacetale, Spiroorthocarbonate, Oxetane oder bicyclische Orthoester einsetzen. Bevorzugte Beispiele sind: 2-Methylen-1,4,6-trioxaspiro[2.2]-nonan, 3,9-Dimethylen-1,5,7,11-tetraoxaspiro[5.5]undecan, 2-Methylen-1,3-dioxepan, 2-Phenyl-4-methylen-1,3-dioxolan, Bisphenol-A-diglycidylether, 3,4-Epoxy-cyclohexylmethyl-3,4-epoxycyclohexancarboxylat, Bis(3,4-epoxycyclohexylmethyl)adipat, Vinylcyclohexendioxid, 3-Ethyl-3-hydroxymethyloxetan, 1,10-Decandiyl-bis-(oxymethylen)-bis-(3-ethyloxetan) oder 3,3-(4-Xylylendioxy)-bis-(methyl-3-ethyl-oxetan) sowie die in der EP 0 879 257 B1 genannten Epoxide. Bevorzugte Monomere sind. Bisphenol-A-diglycidylether, 3,4-Epoxy-cyclohexylmethyl-3,4-epoxycyclohexancarboxylat und Bis(3,4-epoxycyclohexylmethyl)adipat. Als kationisch polymerisierbare Matrixsysteme eignen sich auch Kieselsäurepolykondensate, die kationisch polymerisierbare Gruppen, bevorzugt Epoxid-, Oxetan-, oder Spiroorthoestergruppen tragen, und die beispielsweise durch hydrolytische Kondensation von Silanen zugänglich sind. Bevorzugte Kieselsäurepolykondensate sind solche, die durch hydrolytische Homo- oder Cokondensation von 2-(3,4-Epoxycyclohexyl-trimethoxysilan-und/oder -triethoxysilan erhalten. Weiterhin lassen sich als kationisch polymerisierbare Monomere auch Vinylether, wie z.B. Ethyl- oder Isobutylvinylether, sowie N-Vinylpyrrolidon einsetzen. Als Monomere für die ringöffnende Metathesepolymerisation (RÖMP) lassen sich bekannte RÖMP-Monomere, wie monocyclische Alkene oder Alkadiene, beispielsweise Cyclopenten, Cyclohepten, Cycloocten, Cyclododecen oder 1,5-Cyclooctadien, oder bicyclische Alkene, beispielsweise Bicyclo[2.2.1]hept-2-en(2-Norbornen) oder davon abgeleitete Derivate, wie 7-Oxa-bicyclo[2.2.1]hept-2-en, Bicyclo[2.2.1 ]hept-5-en-2,3-dicarbon-säuredimethylester, 7-Oxabicyclo[2.2.1]hept-5-en-2,3-dicarbon-säurediethylester, 5-Norbornen-2-methylester oder 5-Norbornen-2-yl-ester von Mono-, Di- und Multicarbonsäuren oder die Umsetzungsprodukte von 5-Norbornen-2-methanol oder 5-Norbornen-2-ol mit Mono- oder Diisocyanaten einsetzen. Dabei lassen sich die RÖMP-Monomere z.B. auch in Mischung mit radikalisch polymerisierbaren Monomeren, insbesondere mit mono- oder multifunktionellen (Meth)acrylaten verwenden. Als polyreaktives Bindemittel A eignen sich insbesondere auch Thiol-En-Harze, die aus Mischungen von mono oder multifunktionellen Mercaptoverbindungen und di- oder multifunktionellen ungesättigten Monomeren, vor allem Allyl- oder Norbonenverbindungen bestehen. Beispiele für mono- oder multifunktionellen Mercaptoverbindungen sind o-, m oder p-Dimercaptobenzol und Ester der Thioglykol- oder der 3-Mercaptopropionsäure von Ethylen-, Propylen- oder Butylenglykol, Hexandiol, Glycerin, Trimethylolpropan oder Pentaerythrit. Beispiele für di- oder multifunktionelle Allylverbindungen sind Ester von Allylalkohol mit Di- oder Tricarbonsäuren, wie Malon-, Malein- Glutar-, Bernstein-, Adipin-, Sebacin-, Phthal-, Terephthal- oder Gallussäure sowie mono- oder trifunktionelle Allylether, wie z.B. Diallylether, α,ω-Bis[allyloxy]alkane, Resorcin- oder Hydrochinondiallylether sowie Pyrogalloltriallylether, oder andere ungesättigte Verbindungen wie z.B. 1,3,5-Triallyl-1,3,5-triazin-2,4,6-(1H,3H,5H)- trion, Tetraallylsilan oder Tetraallylorthosilikat. Beispiele für di- odermultifunktionelle Norbonenverbindungen sind Diels-Alder-Additionsprodukte von Cyclopentadien oder Furan mit di- oder multifunktionellen (Meth)acrylaten, sowie Ester bzw. Urethane von 5-Norbornen-2-methanol oder 5-Norbornen-2-ol mit Di- oder Polycarbonsäuren, wie z.B. Malon- Malein-, Glutar-, Bernstein-, Adipin-, Sebacin-, Phthal-, Terephthal- oder Gallussäure, mit bzw. Di-oder Polyisocyanaten, wie Hexamethylendiisocyanat bzw. dessen cyclisches Trimer, 2,2,4-Trimethylhexamethylendiisocyanat, Toluylendiisocyanat oder Isophorondiisocyanat. Dabei zeichnen sich Thiol-En-Harze vorteilhafter Weise durch eine geringe Viskosität und einen geringen Polymerisationsschrumpf aus.

Darüber hinaus führen die Mercaptogruppen der Thiol-Komponente zu einer Wechselwirkung mit der Oberfläche der Keramikpartikel und stabilisieren somit den Schlicker.Besonders bevorzugte Thiol-En-Komponenten sind Mischungen aus Estern der 3-Mercaptopropionsäure mit Hexandiol, Glycerin, Trimethylolpropan und/oder Pentaerythrit mit einer oder mehreren der folgenden En-Komponenten: Bicyclo[2.2.1]hept-2-en, Bicyclo[2.2.1]hept-5-en-2,3-dicarbonsäuredimethylester, 7-Oxa-bicyclo[2.2.1]hept-5-en-2,3-dicarbonsäurediethylester, 5-Norbornen-2-methylester der Bernsteinsäure oder Adipinsäure sowie Ester von Allylalkohol mit Adipin-, Terephthal- oder Gallussäure, Hydrochinondiallylether, Pyrogalloltriallylether und 1,3,5-Triallyl-1,3,5-triazin-2,4,6-(1H,3H,5H)-trion. Als polyreaktives Bindemittel lassen sich auch sog. Michael-Reaktionsharze einsetzen. Dabei handelt es sich vorzugsweise um Mischungen von Acylamiden und/oder Bisacrylamiden, insbesondere di- und/oder multifunktionellen Acrylaten mit di- oder multifunktionellen Acetoacetaten. Beispiele für geeignete Acrylate sind Ethylenglycoldiacrylat, Hexandioldiacrylat, Tripropylenglycoldiacrylat, ethoxyliertes Bisphenol-A-Diacrylat, Polyethylenglycol-200-diacrylat, Trimethylolpropantriacrylat, Pentaerythrittetraacrylat. Diese Acrylate lassen sich insbesondere mit di-, tri- oder tetrafunktionellen Acetoacetaten, wie z.B. Hexandioldiacetoacetat, Trimethylolpropan- und Glycerintrisacetoacetat sowie Pentaerythrittetrakisacetoacetat zu Netzwerkpolymeren umsetzen. Dabei kann die Bildung geeigneter Katalysatoren und damit die Michael-Reaktion zwischen den di-oder multifunktionellen Acrylaten mit den di- oder multifunktionellen Acetoacetaten photochemisch induziert werden. Als Katalysatoren für die Michael-Reaktion sind starke Basen besonders geeignet, wie z.B. 1,5-Diazabicyclo[4.3.0]non-5-en (DBN), die durch Bestrahlen mit Licht aus entsprechenden Precursoren freigesetzt werden können.

Durch die Wahl von Spacergruppen kann die Flexibilität der Polymermatrix beeinflusst werden Unter Spacergruppen - auch Abstandshalter genannt - versteht man Reste, die zwei oder mehrere funktionelle Gruppen, wie z.B. Acetoacetat- oder Acrylat-Gruppen miteinander verbinden. Beispielsweise ergeben längere und/oder unpolare Spacergruppen, wie z.B. Decamethylen-Gruppen, im Vergleich zu Propylen- oder Methylenoxymethylen-Spacern eine höhere Flexibilität. Erfindungsgemäß werden vorzugsweise Mischungen verwendet, die weniger als 20 Gew% Monoacrylate und Monoacetoacetate enthalten. Besonders bevorzugte Photoinitiatoren sind Norrish-Typ-1-Photoinitiatoren, vor allem Monoacyltrialkyl- oder Diacyldialkylgermanium-Verbindungen, wie z.B. Benzoyltrimethylgermanium, Dibenzoyldiethylgermanium oder Bis (4-methoxybenzoyl)diethylgermanium. Dabei lassen sich auch Mischungen der verschiedenen Photoinitiatoren einsetzen, wie z.B. Dibenzoyldiethylgermanium in Kombination mit Campherchinon und 4-Dimethylaminobenzoesäureethylester.

Schlicker auf der Basis von kationisch polymerisierbaren Harzen lassen sich mit den bekannten kationischen Photoinitiatoren, besonders mit Diaryliodonium- oder Triarylsulfoniumsalzen, ggf. in Gegenwart geeigneter Sensibilisatoren, wie z.B. Campherchinon, Monoacyltrialkyl- oder Diacyldialkylgermanium-Verbindungen, wie z.B. Benzoyltrimethylgermanium, Dibenzoyldiethylgermanium oder Bis(4- methoxybenzoyl)diethylgermanium, aushärten. Beispiele für geeignete Diaryliodoniumsalze, die mit Campherchinon, Monoacyltrialkyl- oder Diacyldialkylgermanium-Verbindungen oder Thioxanthonen als Sensibilisator im sichtbaren Bereich eingesetzt werden können, sind die kommerziell zugänglichen 4-Octyloxy-phenylphenyl-iodoniumhexafluoroantimonat oder Isopropylphenyl-methylphenyl-iodoniumtetrakis(pentafluorophenyl)borat. Zur Auslösung der lichtinduzierten Michael-Addition von Schlickern auf der Basis von Michael- Reaktionsharzen lassen sich sog. photolatente Basen einsetzen. Hierbei handelt es sich um Verbindungen, die beim Einstrahlen von Licht katalytisch wirksame tertiäre Aminkomponenten bilden. Beispiele hierfür sind photolatente Amidine, wie z.B. 5-Benzyl-1,5-diazabicyclo[4.3.0]non-5-en, das bei Bestrahlung 1,5-Di-azabicyclo[4.3.0]-5-nonen bildet, welches ein sehr wirksamer Katalysator für die Michael-Reaktion von Acetoacetaten mit Acrylaten ist. Photolatente Basen können auch mit bekannten Sensibilisatoren für den sichtbaren Bereich kombiniert werden. Als Photosensibilisatoren sind α-Diketone, wie 9,10-Phenanthrenchinon, Diacetyl oder besonders Campherchinon bevorzugt. Die Partikelgröße der Photoinitiatoren liegt beispielsweise im Bereich von 50 nm bis 50 Pm. Sie ist abhängig von der eingesetzten Keramik. Bei Al₂O₃ liegt die Größe der als Komponente B verwendeten Partikel vorzugsweise im Bereich von 50 bis 500 nm, besonders bevorzugt zwischen 75 und 200 nm; bei Glaskeramik im Bereich von 500 nm und 50 Pm, ganz bevorzugt zwischen 1 und 10 Pm; bei TZP-3Y Zirkondioxid im Bereich von 50 und 500 nm, ganz bevorzugt zwischen 50 und 350 nm. Die Partikelgröße wird dabei vorzugsweise so gewählt, dass sedimentationsstabile Schlicker erhalten werden. Bei den Partikelgrößen handelt es sich um die absoluten Ober- und Untergrenzen, Als nicht polymerisierbare Oberflächenmodifikatoren eigen sich insbesondere lineare oder verzweigte Carbonsäuren, wie z.B. Ameisensäure, Essigsäure, Propionsäure, Octansäure, Iso-buttersäure, Isovaleriansäure, Pivalinsäure, saure Phosphorsäureester, wie z.B. Dimethyl-, Diethyl-, Dipropyl-, Dibutyl-, Dipentyl-, Dihexyl-, Dioctyl- oder Di(2-ethylhexyl)phosphat, oder Phosphonsäuren, z.B. wie Methyl-, Ethyl-, Propyl-, Butyl-Hexyl, Octyl- oder Phenylphosphonsäure. Als nicht polymerisierbare Oberflächenmodifikatoren geeignete Silane sind beispielsweise Propyltrimethoxysilan, Phenyltrimethoxysilan, Hexyltrimethoxysilan, Octyltrimethoxysilan, Trimethylchlorsilan, Trimethylbromsilan, Trimethylmethoxysilan oder Hexamethyldisilazan. Die Schlicker können auch ein Lösungsmittel enthalten. Als Lösungsmittel eignen sich beispielsweise die oben als Weichmacher genannten Verbindungen. Vorzugsweise werden als Lösungsmittel solche Komponenten eingesetzt, die eine Siedetemperatur von mindestens ca. 120 °C, vorzugsweise von 150 bis 250 °C, besonders bevorzugt von 180 bis 230 °C aufweisen, so dass es bei der stereolithographischen Verarbeitung des Schlickers nicht zu einer vorzeitigen Verdunstung kommt. Dabei sind Mischungen von solchen Lösungsmitteln besonders geeignet, die in einem Temperaturbereich zwischen 150 und 250 °C schrittweise verdampfbar sind. Ganz besonders geeignet sind Octanol, Triethylenglycol-Divinylether, 2-Amino-2- methyl-1-propanol, 2-Methyl-2,4-pentandiol, Ammoniumcitrat tribasisch (fest), Tripropylenglycol, Tetraethylenglycol, Triethylenglycol, Triethylcitrat, Acet-Essigsäure-Ethylester, Cyclohexanol, Cyclohexanon, Diethylenglycolmonomethylether, Oxalsäuredibutylester, 2.5-Dimethoxyterahydrofuran, Polythylenlycol 300, 1-oder 2-Nonanol, Diethylenglycoldiethylether, 2,5-Dimethoxytetrahydrofuran, Oxalsäuredibutylester, Cyclohexanol, Cyclohexanon, Acetessigsäureethylester und Mischungen davon. Das oder die Lösungsmittel werden vorzugsweise in einer Menge von 5 und 50 Gew%, besonders bevorzugt von 10 und 30 Gew% eingesetzt, bezogen auf die Masse des polyreaktiven Bindemittels.

Die Schichtdicke der jeweiligen Schicht bei der schichtweisen Herstellung des Vorformlings hängt von der in der Suspension (Bindemittel) absorbierten Energie während der Belichtung ab, die im Falle einer gepulsten Lichtquelle beispielsweise durch die Anzahl der Pulse, die Energiedichte pro Puls, die Puls-Rate pro Zeiteinheit und die Wellenlänge des Belichtungslichts beeinflusst wird. Als Lichtquelle für den Stereolithographie-Prozess kann beispielsweise ein Argon- Laser verwendet werden. Ein biokompatibler Binder ist beispielsweise ein OrmoComp-Hybridpolymer, das unter UV-Licht aushärtet. Der Aushärtungsprozess der belichteten Schicht läuft dabei mit gesteigerter Intensität der Lichtquelle schneller ab. Während dieser beispielsweise bei einer Intensität von 2mW/cm² eine Aushärtezeit im Minutenbereich benötigt, kann die Aushärtung bei einer Intensität von über 10mW/cm² in den Sekundenbereich verschoben werden. Durch die Lichtabsorption der Magnesium- oder Titan-Partikel in der Suspension wird eine höhere Lichtintensität als bei anderen nicht absorbierenden Leichtmetallpulvern benötigt werden.

Die Verwendung von Stereolithographie zur Herstellung des Vorformlings stellt zudem eine flexible und leicht zu variierende Produktionsmethode dar, die eine kostengünstige und hochvariable Herstellung individueller Leichtmetallbauteile ermöglicht, wobei die Leichtmetallbauteile sehr unterschiedliche und hochkomplexe Strukturen besitzen können. Solche Leichtmetallbauteile sind zudem innerhalb weniger Stunden bedarfsgerecht produzierbar, je nach Bedarf auch unter sterilen Bedingungen, was gerade für Implantate wichtig ist. Stereolithographische Anlagen und Backöfen können als sehr kompakte Komponenten bereitgestellt werden, sodass sich das erfindungsgemäße Verfahren auch in einer mobilen Produktionsstätte lokal am Ort des Bedarfs anwendbar ist, was geringe Reaktionszeiten und vermiedene Transportstrecken bei der Bereitstellung der Leichtmetallbauteile ermöglicht.

Als Vorformling wird dabei ein noch ungebackener Rohling umfassend die noch unverbundenen oder nicht stabil verbundenen Pulvermaterialien zur späteren Herstellung eines verbackenen Körpers bezeichnet. Der Vorformling lässt sich bei Bedarf noch leicht bearbeiten. Der Vorformling ist in diesem Verfahren ein Bauteil aus einem mit dem Bindemittel verklebten Pulver. Der Stereolithographie-Prozess ist dabei so ausgelegt, dass der dadurch entstehende Vorformling so bemessen ist, dass dieser durch das Schwinden beim Backen seine die endgültige Form erhält. Durch das Backen wird der Vorformling auf eine Temperatur geeignet zum Aufbrechen einer Oxydschicht des Pulvermaterials erhitzt, die bei Titan bei oberhalb 400°C liegt, bei Temperaturen oberhalb von 650°C brechen die Oxidschicht bei Magnesium auf. Diese Temperaturen sind geeignete Temperaturen im Sinne der Erfindung. Hierbei wird die geeignete Temperatur auf ein Temperaturintervall nach oben so begrenzt, dass die Leichtmetallpulver nicht oder zumindest nicht vollständig aufgeschmolzen werden. Bei Titan oder Titanoxid als Pulvermaterial ist eine Backtemperatur bis knapp unterhalb der jeweiligen Schmelztemperatur von 1668°C beziehungsweise 1843°C möglich, wobei zur vereinfachten Prozessführung bei Titanmaterialien vorteilhafterweise Backtemperaturen zwischen 1000°C und 1400°C verwendet werden. Magnesium besitzt dagegen eine Schmelztemperatur von 650°C. Daher kann der Teil des Magnesiumpulvers, der bereits reduziert wurde, während des Backvorgangs zumindest zum Teil angeschmolzen werden, daher sollte hier die Backtemperatur nicht zu hoch gewählt werden. Sofern Magnesiumoxid mit einer Schmelztemperatur von 2852°C als Pulvermaterial verwendet wird, kann zumindest am Anfang des Backvorgangs eine deutlich höhere Backtemperatur verwendet werden als bei reinem Magnesiumpulver. Die Backtemperatur ist dabei so bemessen, dass die grundsätzliche Gestalt des Bauteils erhalten bleibt. Dabei kommt es in der Regel zu einer Schwindung, weil sich die Partikel des Magnesium- oder Titanpulvers verdichten und Porenräume aufgefüllt werden, während das organische Bindermaterial vollständig oder zumindest teilweise ausgetrieben wird. Das Austreiben des Bindemittels setzt in der Regel schon bei Temperaturen unterhalb der Backtemperatur beziehungsweise der Sintertemperatur ein, beispielsweise bei 350°C bis 500°C. Vorteilhafterweise wird daher beim Backprozess das Temperaturintervall unterhalb der gewünschten Backtemperatur schnell zur Erreichung der Backtemperatur durchfahren. Beim Austreiben des Binders finden rein thermische wie auch thermochemische Prozesse statt. Üblicherweise werden beim Verpressen von keramischen Pulvern als Binder Mischungen von Wasser, Lösungsmitteln, Polymeren, Wachsen oder Ölen eingesetzt. Als Polymere finden meist Polypropylen, Polyethylen, Polyvinylacetat, Polyvinylalkohol, Methylcellulose, Polyvinylpyrrolidon, Polystyrol oder Polyethylmethacrylat Anwendung. Hierbei handelt es sich um lineare Polymere, die durch Depolymerisation oder Kettenspaltung bei höherer Temperatur mehr oder weniger leicht zu flüchtigen Komponenten abgebaut werden. Die Festigkeit des gebackenen Vorformlings wird unter anderem durch ausgebildete Hälse zwischen den Pulverpartikeln erreicht, die sich mittels Oberflächendiffusion zwischen den Pulverpartikeln bilden. Das vorher miteinander verklebte Pulvermaterial wird durch die Erwärmung miteinander verbunden und verdichtet. Im Gegensatz zur reinen Schmelze werden hierbei jedoch die Körner des Pulvermaterials nicht oder zumindest nicht vollständig aufgeschmolzen. Das Pulvermaterial im Vorformling wird durch das Backen somit zusammengebacken. Das erfindungsgemäße Verfahren macht es sich zunutze, dass gerade Pulver eine große Oberfläche und damit eine große Oberflächenenergie besitzen. Da jedes System danach strebt, einen Zustand geringster freier Enthalpie einzunehmen, vergrößern sich die einzelnen Pulverkörner, so dass die Oberflächenenergie sinkt. Damit steigt der Anteil abgesättigter chemischer Bindungen, so dass sich der Körper insgesamt zum gewünschten Leichtmetallbauteil verfestigt.

In einem anderen Beispiel enthält der Vorformling einen sogenannten Entbinderungsbeschleuniger zum Austreiben des Binders. Dieser wird vorzugsweise in einer Menge von 0 bis 20 Gew%, besonders bevorzugt 0,01 bis 10 Gew% eingesetzt, jeweils bezogen auf die Gesamtmasse des Schlickers. Unter Das Austreiben des Binders aus dem des Grünkörper kann durch geeignete, d.h. polyreaktionswirksame Stoffe im Polyreaktionsharz gefördert oder zielgerichtet beeinflusst werden. Dabei handelt es sich einerseits um Additive, die die Netzwerkbildung beeinflussen, wie vor allem kettenübertragungsaktive Stoffe, sog. Kettenregler, die zu einer Verringerung der Polymernetzwerkdichte und damit zu einer besseren thermischen Abbaubarkeit führen. Bekannte Kettenregler z.B. für die radikalische Polymerisation sind vor allem Mercaptane, wie z.B. Laurylmercaptan, und Disulfide. Disulfide, vor allem Dithiourethandisulfide, wie z.B. Tetramethylthiuramdisulfid oder Isopropylxanthogensäuredisulfid wirken bei der radikalischen Photopolymerisation als sog. Photoiniferter. Es handelt sich dabei um Verbindungen, die sowohl als Photoinitiator (Photoini-) wirken, als auch an Übertragungsreaktionen und Abbruchsreaktionen teilnehmen. Die Zugabe von kettenübertragungsaktiven Stoffen, d.h. von Kettenreglern oder Photoinifertern, bewirkt eine Verringerung der Netzwerkdichte des Polyreaktionsnetzwerkes, bei nahezu unveränderter Reaktivität der Polyreaktionsharzmischung. Kettenregler und Photoiniferter werden vorzugsweise in einer Menge von jeweils 0,005 bis 2 Gew% und besonders bevorzugt 0,01 bis 1,0 Gew% bezogen auf die Komponente (A) eingesetzt. Als Entbinderungsbeschleuniger lassen sich auch Comonomere einsetzen, die zu einer Verringerung der thermischen Stabilität von Polymernetzwerken führen. Hierzu eignen sich Comonomere, die thermisch labile Gruppen enthalten, wie z.B. Peroxid-, Azo- oder Urethangruppen, welche während des stereolithographischen Prozesses in das Polymernetzwerk eingebaut werden und dann im thermischen Entbinderungsprozess den Abbau des Polymernetzwerkes beschleunigen. Ein bevorzugtes Beispiel für ein polymerisationsfähiges Peroxid ist 4,4'-Divinylbenzoylperoxid, das durch Umsetzung von 4-Vinylbenzoylchlorid mit Natriumperoxid zugänglich ist. Ein bevorzugtes Beispiel für eine polymerisationsfähige Azo-Verbindung ist der Ester aus 2-Hydroxyethylmethacrylat und der 4,4'-Azobis-(4-cyano-valeriansäure). Bevorzugte thermisch labile Urethane sind aus Diisocyanaten zugänglich, beispielsweise durch Umsetzung von 2,2,4-Trimethylhexamethylendiisocyanat (TMDI) oder Toluylendiisocyanat (TDI) mit Hydroxypropylacrylat (HPA) oder 2-Hydroxyethylacrylat (HEA). Ein weiteres Beispiel für einen thermisch labilen Monomerbaustein stellt α,α,α',α'-Tetramethyl-1,4-benzen-dimethylacrylat dar, dessen Einbau in ein Michael-Addition-Netzwerke beispielsweise von Diacrylaten und Diacetoacetaten in Gegenwart katalytischer Säuremengen zu einem beschleunigten Abbau des Polymernetzwerks führt. Außerdem sind als Entbinderungsbeschleuniger Comonomere geeignet, deren Polyreaktionsprodukte thermisch leicht abbaubar sind. Für radikalische Polymerisationsharze sind Comonomere bevorzugt, die wie a-Methylstyrol eine niedrige ceiling-Temperatur Tc aufweisen. Die ceiling-Temperatur ist die Grenztemperatur, bei der die Polymerisation mit der Depolymerisation im Gleichgewicht steht, und lässt sich aus dem Quotienten der Polymerisationsenthalpie und der Polymerisationsentropie berechnen. Beispielsweise beträgt Tc für α-Methylstyrol 61 °C. Die ceiling-Temperatur TC von Polytetrahydrofuran (PTHF) liegt bei 80 °C. Dementsprechend kann z.B. durch Verwendung von Telechelen, insbesondere PTHF-Di(meth)acrylat-Telechelen als Comonomer die Abbaubarkeit von Poly(meth)acrylatnetzwerken beschleunigt werden. Erfindungsgemäß sind Comonomere mit einer ceiling-Temperatur von -10 bis 150 °C, vorzugsweise 10 bis 150 °C und besonders bevorzugt 20 bis 130°C besonders geeignet. Die Comonomere werden vorzugsweise in einer Menge von 0,1 bis 30 Gew% und besonders bevorzugt 0,5 bis 20 Gew% bezogen auf das polyreaktive Bindemittel eingesetzt.

In einer Ausführungsform des Verfahrens umfasst die Suspension ein wasserfreies Lösungsmittel, vorzugsweise Alkohol, zur Einstellung der Viskosität der Suspension für den Schritt des Herstellens. Mit dieser Suspension wären sowohl Magnesium- als auch Titanmaterialien mit der gleichen Suspension bearbeitbar.

In einer Ausführungsform des Verfahrens wird der Schritt des Backens unter Schutzgas, vorzugsweise Stickstoff oder Argon, oder im Vakuum, vorzugsweise unter 1mbar, ausgeführt. Damit können gezielt nicht-oxidierte Mg-/Ti-Leichtmetallbauteile für die jeweilige Anwendung hergestellt werden. Sofern für eine Anwendung eine bestimmte Oxidschicht auf dem erfindungsgemäßen Leichtmetallbauteil gewünscht oder benötigt ist, kann diese mittels eines oxidierenden Verfahrens gezielt und definiert auf dem bisher nicht-oxidierten Leichtmetallbauteil erzeugt werden. Das Backen des Vorformlings unter Vakuum beeinflusst ebenfalls den Back- beziehungsweise den Sinterprozess, sodass unter Vakuum beispielsweise um 100°C bis 300°C niedrigere Backtemperaturen verwendet werden können als bei Normaldruck.

In einer weiteren Ausführungsform umfasst das Verfahren einen zusätzlichen Schritt des oberflächlichen Oxidierens zur Erzeugung einer Oberflächenoxidschicht auf dem Leichtmetallbauteil, vorzugsweise mit einer Dicke zwischen 2µm und 500µm, besonders bevorzugt von 5 - 20µm, beispielsweise mit einer mittleren Dicke von 10µm. Hiermit kann gezielt und definiert eine Oxidschicht auf dem Leichtmetallbauteil erzeugt werden. Das oberflächliche Oxidieren kann dabei mittels Anodisation oder plasmaelektrolytischer Oxidation durchgeführt werden.

Das Magnesium- und/oder Titanpulver kann beispielsweise mittels Verdüsung hergestellt werden. Hierbei wird das zu verdüsende Material aufgeschmolzen und ein Gasstrahl durch die Schmelze durchgeleitet, wodurch das Material der Schmelze atomisiert wird. Das wieder aufgefangene Material liegt dann in Pulverform vor und kann für das erfindungsgemäße Verfahren verwendet werden. Alternativ kann das Pulvermaterial auch mittels anderer Prozesse gewonnen werden.

In einer Ausführungsform umfasst das Verfahren zumindest nach dem Schritt des Backens beziehungsweise nach dem Schritt des oberflächlichen Oxidierens des Leichtmetallbauteils ein Bereitstellen des Leichtmetallbauteils unter sterilen Bedingungen. Dadurch kann das Leichtmetallbauteil sofort als Implantat verwendet werden, ohne dass zusätzliche Sterilisationsschritte notwendig werden.

### Kurze Beschreibung der Abbildungen

Diese und andere Aspekte der Erfindung werden im Detail in den Abbildungen wie folgt gezeigt:
- Fig. 1:: zwei Ausführungsformen des erfindungsgemäßen strukturierten Leichtmetallbauteils (a) als netzartige Struktur und (b) als Volumennetz;
- Fig. 2:: weitere Ausführungsformen (a) - (d) des erfindungsgemäßen strukturierten Leichtmetallbauteils mit unterschiedlichen netzartigen Strukturen;
- Fig.3:: eine weitere Ausführungsform des erfindungsgemäßen strukturierten Leichtmetallbauteils mit einem Volumennetz (a) in perspektivischer Darstellung und (b) als Ausschnitt des Volumennetz;
- Fig.4:: ein Schnitt durch eine Strebe des erfindungsgemäßen strukturierten Leichtmetallbauteils;
- Fig.5:: eine Ausführungsform des erfindungsgemäßen Verfahrens zur Herstellung des strukturierten Leichtmetallbauteils.

### Detaillierte Beschreibung der Ausführungsbeispiele

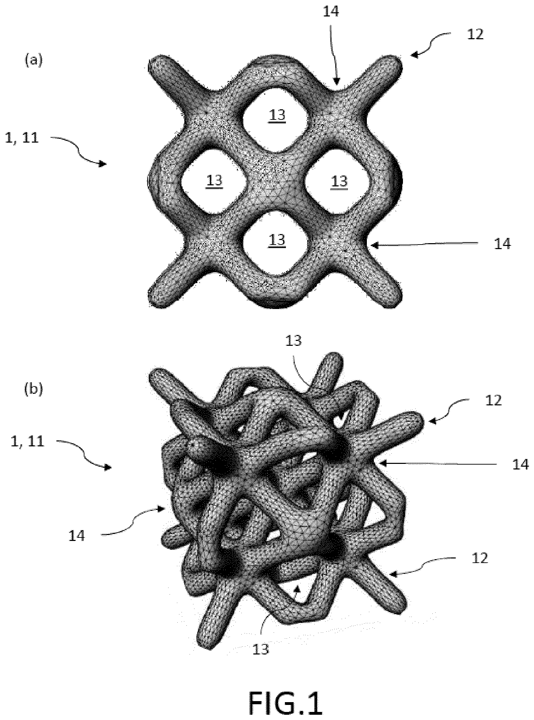

Fig.1 zeigt zwei Ausführungsformen des erfindungsgemäßen strukturierten Leichtmetallbauteils 1 (a) als netzartige Struktur und (b) als Volumennetz. Das strukturierte Leichtmetallbauteil 1 besteht hier zumindest zum überwiegenden Teil aus Magnesium und/oder Titan, wodurch es zum Einsatz als Implantat grundsätzlich geeignet ist, obwohl das Leichtmetallbauteil separate dazu in nichtmedizinischen Anwendungen auch eingesetzt werden kann. Das Leichtmetallbauteil 1 ist dabei aus einem Pulvermaterial 2 umfassend Magnesium und/oder Titan mittels Stereolithographie SL zu einem vorstrukturierten Vorformling 3 und mittels eines geeigneten Backprozesses oberhalb einer Temperatur TB geeignet zum Aufbrechen einer Oxydschicht des Pulvermaterials 2 zu dem Leichtmetallbauteil 1 gefertigt, wodurch das Material des fertigen strukturierten Leichtmetallbauteils 1 zumindest zum überwiegenden Teil aus Magnesium und/oder Titan besteht. Da die Struktur und die Materialeigenschaften neben der Materialwahl Magnesium- oder Titanpulver hauptsächlich durch die Prozessführung bestimmt wird, ist die Charakterisierung des Leichtmetallbauteils sinnvoll nur über die Spezifizierung des Prozesses möglich. Das hier gezeigte Leichtmetallbauteil 1 weist eine netzartige Struktur 11 aus Streben 12 aus dem Material des Leichtmetallbauteils 1 und dazwischen vorhandene Leerräume 13 auf, wobei in Fig.1 (a) das Leichtmetallbauteil 2 eine flächenartige Struktur besitzt, während die in Fig.1(b) gezeigte Struktur eine Volumenstruktur darstellt. Die gezeigten netzartigen Strukturen 11 weisen mehrere Kreuzungs- und/oder Verbindungspunkte 14 der Streben 12 miteinander auf, beim Volumennetz 11 in Fig.1 (b) auch das Volumens einer Einhüllenden um die äußere Ebene dieser Struktur 11 durchdringend. Das Material des Leichtmetallbauteils 1 kann dabei aus Magnesium oder einer Magnesiumlegierung bestehen, die bis zu 1,5% Zink, bis zu 1,5% Calcium, bis zu 5% Yttrium und/oder bis zu 4% seltene Erden umfasst. Beispielweise können Magnesiumlegierungen mit einem Anteil von 4% Yttrium und 3% Seltene Erden ohne Zink und Kalzium (sogenanntes WE43) oder mit einem Anteil von 1% Zink und 1% Kalzium ohne Yttrium und Seltene Erden (sogenanntes ZX11) verwendet werden. Das Material kann eine Materialdichte von mehr als 90%, vorzugsweise mehr als 97%, besonders bevorzugt mehr als 99%, der nominalen Dichte vom jeweiligen Material des Leichtmetallbauteils (überwiegend oder vollständig aus Magnesium oder Titan) aufweisen.

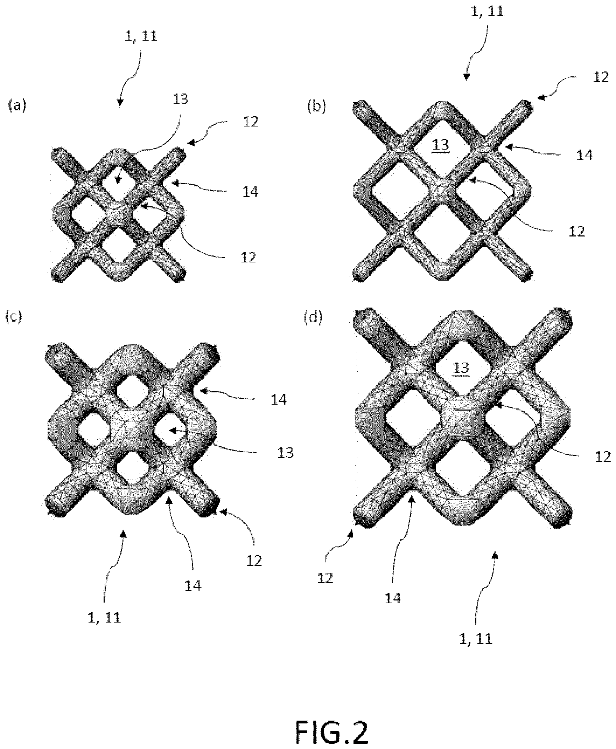

Fig.2 zeigt weitere Ausführungsformen (a) - (d) des erfindungsgemäßen strukturierten Leichtmetallbauteils 1 mit unterschiedlichen netzartigen Strukturen 11. Die Streben 12 können dabei einen Durchmesser von 100µm bis 1500µm und/oder die Leerräume 13 einen Durchmesser zwischen 200µm und 1500µm besitzen. Die Ausführungsform (a) zeigt eine Struktur mit mittleren Durchmessern für Streben 12 und Leerräume 13. Die Ausführungsform (b) zeigt eine Struktur mit einem mittleren Durchmesser für die Streben 12 und einen großen Durchmesser für die Leerräume 13. Die Ausführungsform (c) zeigt eine Struktur mit einem großen Durchmesser für die Streben 12 und einen kleinen Durchmesser für die Leerräume 13. Die Ausführungsform (d) zeigt eine Struktur mit großen Durchmessern für die Streben 12 und für die Leerräume 13.

Fig.3 zeigt eine weitere Ausführungsform des erfindungsgemäßen strukturierten Leichtmetallbauteils 1 mit einem Volumennetz 11 (a) in perspektivischer Darstellung und (b) als Ausschnitt des Volumennetzes 11. Der Ausschnittbereich ist in Fig3a durch das weiße Quadrat bezeichnet und mit den gestrichelten Linien zur Verdeutlichung mit Fig.3b verbunden. Das Leichtmetallbauteil 1 in Fig.3a umfasst hier einen ersten Bereich 1a, in dem das Volumennetz 11 angeordnet ist, und zwei zweite Bereiche 1b auf beiden Seiten des ersten Bereichs 1a, in denen jeweils eine kompakte Struktur 18 angeordnet ist. Die kompakten Strukturen 18 sind dabei durch den Herstellungsprozess fest mit der netzartigen Struktur 11 verbunden. Die kompakten Strukturen 18 können beispielsweise der Befestigung des Leichtmetallbauteils 1 auf einer dafür vorgesehenen Unterlage (hier nicht gezeigt) dienen. Dafür weisen die kompakten Strukturen 18 hier beispielsweise Löcher 18a auf, durch die Schrauben zur Befestigung hindurchgesteckt werden können. Im Falle eines Implantats könnte die Unterlage Knochenmaterial sein. Eine solche Struktur 11 ist sowohl als Implantat 1 zum Knochenersatz als auch als Leichtmetallbauteil 1 in der Automobilindustrie (beispielsweise als Knautsch- oder Verstärkungszone) oder in der Luft- und Raumfahrtindustrie als leichte Trägerstruktur für weitere Komponenten geeignet. Das Leichtmetallbauteil kann in anderen Ausführungsformen auch weitere dritte, vierte oder weitere Bereiche 1a, 1b umfassen, in denen je nach Anwendung netzartige Strukturen 11 oder kompakte Strukturen 18 angeordnet sind. Das in Fig.3b als Ausschnitt aus Fig.3a gezeigte Volumennetz 11 aus Streben 12 und Leerräumen 13 dazwischen besitzt eine Strukturdichte über das Volumen in ersten Bereich 1a von 25% im Vergleich zu einem kompakten Körper, wobei die Strukturdichte durch andere Streben-Durchmesser und Leeraumgrößen je nach Anwendung kleiner oder größer eingestellt werden kann. Die Streben 12 und Leerräume 13 sind hier beispielsweise so angeordnet, dass die netzartige Struktur 11 einen Lichtkanal 19 aufweist, durch den externes Licht das Leichtmetallbauteil 1 passiert. In den anderen Bereichen des Volumennetzes 11 sind die Streben 12 und Leerräume 1 so angeordnet, dass in der hier gezeigten Orientierung des Leichtmetallbauteils 1 kein Licht durch das Volumennetz 11 hindurchdringt, wodurch diese Passagen dunkel dargestellt sind. Dieser Lichtkanal 19 kann je nach Ausführungsform eine beliebige Richtung durch das Leichtmetallbauteil 1 aufweisen. Das Leichtmetallbauteil 1 ist in dieser Ausführungsform nicht opak, also lichtdurchlässig. Die netzartige Struktur 11 kann in anderen Ausführungsformen weitere Lichtkanäle 19 gebildet durch die entsprechend ausgerichtete Anordnung der Streben 12 und Leerräume 13 aufweisen.

Fig.4 zeigt einen Schnitt durch eine Strebe 13 des erfindungsgemäßen strukturierten Leichtmetallbauteils 1. Der Kern der Strebe 13 besteht aus einem Material zumindest zum überwiegenden Teil aus Magnesium und/oder Titan. In dieser Ausführungsform weist das Leichtmetallbauteil 1 eine Oberflächenoxidschicht 15 auf, beispielsweise indem das Material des Leichtmetallbauteils 1 beziehungsweise der hier gezeigten Strebe 13 oberflächlich oxidiert wurde. In dieser Ausführungsform weist das Leichtmetallbauteil 1 zusätzlich eine antibakterielle Beschichtung 16 oder ein Antibiotikum 16' zusätzlich außen auf der Oxidschicht 15 auf. Das Leichtmetallbauteil 1 kann zur Erzeugung einer antibakteriellen Wirkung aber auch selber einen Anteil von bis zu 10% Silber umfassen. Damit ist das Leichtmetallbauteil 1 in der Anwendung als Implantat besonders geeignet. Das Leichtmetallbauteil 1 kann stattdessen oder zusätzlich eine Beschichtung 17 aus einem Material der Gruppe nativer oder künstlicher Kollagene, Fibrine, natürlicher oder künstlicher Seiden, natürlicher Proteine oder Polymere wie PLA, PDLA, PLLA, PTFE oder einer Sol-Gel-Beschichtung, aufweisen. Falls die Beschichtung 17 in Verbindung mit einer Beschichtung 16, 16' verwendet wird, ist die Beschichtung 17 unterhalb der Beschichtung 16, 16' angeordnet.

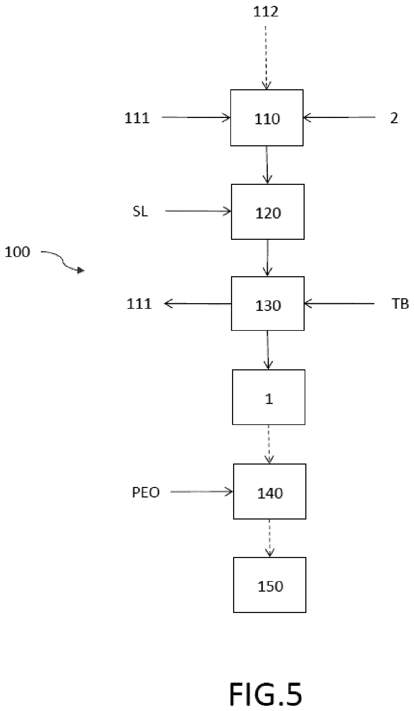

Fig.5 zeigt eine Ausführungsform des erfindungsgemäßen Verfahrens 100 zur Herstellung des erfindungsgemäßen strukturierten Leichtmetallbauteils 1 aus einem Material geeignet zum Einsatz als Implantat. Das Verfahren umfassend die Schritte des Bereitstellens einer Suspension 110 umfassend zumindest ein organisches Bindemittel 111, ein wasserfreies Lösungsmittel 112 (vorzugsweise Alkohol) zur Einstellung der Viskosität der Suspension 110 sowie ein Pulvermaterial 2 umfassend Magnesium und/oder Titan in einer geeigneten Konzentration und Pulvergröße, vorzugsweise mit einem Durchmesser kleiner 200µm (beispielsweise größer als 100µm oder 1µm bis 100µm, insbesondere 20µm-63µm); des schichtweisen Herstellens 120 eines vorstrukturierten Vorformlings 3 aus der Suspension mittels Stereolithographie SL mit einer Energiedichte, die geeignet ist, dass das Bindemittel 111 das Pulvermaterial 2 miteinander zu einer stabilen Vorstruktur verbindet; und des Backens 130 des Vorformlings 2 bei einer geeigneten Temperatur TB geeignet zum Aufbrechen einer Oxydschicht des Pulvermaterials 2 und zur Verbindung des Leichtmetallmaterials 2 der Vorstruktur unter zumindest teilweisem Austrieb des Bindemittels 111 zum fertigen strukturierten Leichtmetallbauteil 1, das nach erfolgtem Backen zumindest zum überwiegenden Teil aus Magnesium und/oder Titan besteht. Der Schritt des Backens 130 kann dabei unter Schutzgas, vorzugsweise Stickstoff oder Argon, oder im Vakuum, vorzugsweise unter 1mbar, ausgeführt werden. Das Verfahren umfassend hier zusätzlich den Schritt des oberflächlichen Oxidierens 140 zur Erzeugung einer Oberflächenoxidschicht 15 auf dem Leichtmetallbauteil 1, vorzugsweise mit einer Dicke zwischen 2µm und 500µm, besonders bevorzugt von 5 - 20µm. Beispielsweise kann eine Oxidschicht eine mittlere Dicke von 10µm besitzen. Hierbei kann der Schritt des oberflächlichen Oxidierens 140 mittels Anodisation oder plasmaelektrolytische Oxidation PEO durchgeführt werden. In einer Ausführungsform umfasst das Verfahren hier nach dem Schritt des oberflächlichen Oxidierens 140 des Leichtmetallbauteils 1 ein Bereitstellen 150 des Leichtmetallbauteils 1 unter sterilen Bedingungen. Dadurch kann das Leichtmetallbauteil 1 sofort als Implantat verwendet werden, ohne dass zusätzliche Sterilisationsschritte notwendig werden.

Je nach Gestaltung der Unterlage zum schichtweisen Herstellen 120 eines vorstrukturierten Vorformlings 3 aus der Suspension mittels Stereolithographie SL und der Belichtungsanordnung (hier nicht gezeigt) in der Stereographie-Anordnung können in demselben Stereolithographie-Prozess auch mehrere Vorformlinge 3 parallel zueinander auf derselben Unterlage oder mit mehreren gleich prozessierten Unterlagen hergestellt werden. Im Backprozess können je nach Größe der Vorformlinge 3 und des Backofens (hier nicht explizit gezeigt) auch mehrere Vorformlinge 3 parallel zueinander in demselben Backofen gebacken 130 werden.

Die hier gezeigten Ausführungsbeispiele stellen nur Beispiele für die vorliegende Erfindung dar und dürfen daher nicht einschränkend verstanden werden. Alternativen durch den Fachmann in Erwägung gezogene Ausführungsformen sind gleichermaßen vom Schutzbereich der vorliegenden Erfindung umfasst.

### Liste der Bezugszeichen

- 1: erfindungsgemäßes Leichtmetallbauteil
- 1a: erster Bereich des Leichtmetallbauteils (netzartige Struktur)
- 1b: zweiter Bereich des Leichtmetallbauteils (kompakte Struktur)
- 11: netzartige Struktur des Leichtmetallbauteils
- 12: Streben der netzartigen Struktur
- 13: Leerräume zwischen den Streben
- 14: Knoten- oder Verbindungspunkte der Streben in der netzartigen Struktur
- 15: Oberflächenoxidschicht
- 16, 16': antibakterielle Beschichtung beziehungsweise Antibiotikum
- 17: äußere (gegebenenfalls weitere) Beschichtung des Leichtmetallbauteils
- 18: kompakte Struktur des Leichtmetallbauteils
- 18a: Löcher (oder Bohrungen) in der kompakten Struktur
- 19: Lichtkanal
- 2: Pulvermaterial
- 3: Vorformling

- 100: erfindungsgemäßes Verfahren
- 110: Bereitstellen einer Suspension für den nachfolgenden Schritt 120
- 111: Bindemittel
- 112: wasserfreies Lösungsmittel
- 110: Bereitstellen einer Suspension für den nachfolgenden Schritt 120
- 120: schichtweises Herstellen eines vorstrukturierten Vorformlings aus der Suspension mittels Stereolithographie
- 130: Backen des Vorformlings
- 140: oberflächliches Oxidieren des Leichtmetallbauteils
- 150: Bereitstellen des Leichtmetallbauteils unter sterilen Bedingungen

- PEO: Anodisation oder plasmaelektrolytische Oxidation
- SL: Stereographie
- TB: geeignete Temperatur für den Backprozess

## Patentansprüche

1. Ein strukturiertes Leichtmetallbauteil (1) aus einem Material vorzugsweise geeignet zum Einsatz als Implantat, das aus einem Pulvermaterial (2) umfassend Magnesium und/oder Titan mittels Stereolithographie (SL) zu einem vorstrukturierten Vorformling (3) und mittels eines geeigneten Backprozesses oberhalb einer Temperatur geeignet zum Aufbrechen einer Oxydschicht des Pulvermaterials zu dem Leichtmetallbauteil (1) gefertigt ist und das Material des fertigen strukturierten Leichtmetallbauteils (1) zumindest zum überwiegenden Teil aus Magnesium und/oder Titan besteht,
**dadurch gekennzeichnet,**
**dass** das Leichtmetallbauteil (1) eine netzartige Struktur (11) aus Streben (12) aus dem Material des Leichtmetallbauteils (1) und dazwischen vorhandene Leerräume (13) umfasst, vorzugsweise ist die netzartige Struktur (11) als Volumennetz ausgebildet, und
wobei die netzartige Struktur (11) mehrere Kreuzungs- und/oder Verbindungspunkte (14) der Streben miteinander aufweist.

2. Das Leichtmetallbauteil (1) nach Anspruch 1,
**dadurch gekennzeichnet,**
**dass** die Streben (12) einen Durchmesser von 100µm bis 1500µm und/oder die Leerräume (13) einen Durchmesser zwischen 200µm und 1500µm besitzen.

3. Das Leichtmetallbauteil (1) nach einem der voranstehenden Ansprüche,
**dadurch gekennzeichnet,**
**dass** das Leichtmetallbauteil (1) mindestens einen ersten Bereich (1a) und einen zweiten Bereich (1b) umfasst, wobei die netzartige Struktur (11) im ersten Bereich (1a) und eine kompakte Struktur (18) zumindest im zweiten Bereich (1b) angeordnet ist.

4. Das Leichtmetallbauteil (1) nach einem der voranstehenden Ansprüche,
**dadurch gekennzeichnet,**
**dass** das Material des Leichtmetallbauteils (1) eine Oberflächenoxidschicht (15) aufweist.

5. Das Leichtmetallbauteil (1) nach einem der voranstehenden Ansprüche,
**dadurch gekennzeichnet,**
**dass** das Leichtmetallbauteil (1) ein Implantat ist, vorzugsweise weist das Leichtmetallbauteil (1) zusätzlich eine Beschichtung (16) mit antibakterieller Wirkung oder ein Antibiotikum (16') auf oder umfasst einen Anteil von bis zu 10% Silber.

6. Das Leichtmetallbauteil (1) nach einem der vorherigen Ansprüche,
**dadurch gekennzeichnet,**
**dass** das Leichtmetallbauteil (1) zusätzlich eine Beschichtung (17) aus einem Material der Gruppe nativer oder künstlicher Kollagene, Fibrine, natürlicher oder künstlicher Seiden, natürlicher Proteine oder Polymere wie PLA, PDLA, PLLA, PTFE oder einer Sol-Gel-Beschichtung, aufweist.

7. Das Leichtmetallbauteil (1) nach einem der vorherigen Ansprüche,
**dadurch gekennzeichnet,**
**dass** das Material aus Magnesium oder einer Magnesiumlegierung besteht, die bis zu 1,5% Zink, bis zu 1,5% Calcium, bis zu 5% Yttrium und/oder bis zu 4% seltene Erden umfasst.

8. Das Leichtmetallbauteil (1) nach einem der Ansprüche 1 bis 6,
**dadurch gekennzeichnet,**
**dass** das Material aus Titan oder einer Titanlegierung besteht, wobei der Backprozess ein Sinterprozess ist und die geeignete Temperatur zwischen 400 Grad und der Schmelztemperatur von Titan liegt.

9. Das Leichtmetallbauteil (1) nach einem der vorherigen Ansprüche,
**dadurch gekennzeichnet,**
**dass** das Material eine Materialdichte von mehr als 90%, vorzugsweise mehr als 97%, besonders bevorzugt mehr als 99%, derjeweiligen nominalen Dichte von Magnesium oder Titan aufweist.

10. Ein Verfahren (100) zur Herstellung eines strukturierten Leichtmetallbauteils (1) aus einem Material geeignet zum Einsatz als Implantat nach Anspruch 1 umfassend die Schritte
• Bereitstellen einer Suspension (110) umfassend zumindest ein organisches Bindemittel (111) sowie ein Pulvermaterial (2) umfassend Magnesium und/oder Titan in einer geeigneten Konzentration und Pulvergröße, vorzugsweise mit einem Durchmesser kleiner 200µm;
• schichtweises Herstellen (120) eines vorstrukturierten Vorformlings (3) aus der Suspension mittels Stereolithographie (SL) mit einer Energiedichte, die geeignet ist, dass das Bindemittel (111) das Pulvermaterial (2) miteinander zu einer stabilen Vorstruktur verbindet;
und
• Backen (130) des Vorformlings (2) bei einer geeigneten Temperatur (TB) geeignet zum Aufbrechen einer Oxydschicht des Pulvermaterials und zur Verbindung des Leichtmetallmaterials (2) der Vorstruktur unter zumindest teilweisem Austrieb des Bindemittels (111) zum fertigen strukturierten Leichtmetallbauteil (1), das nach erfolgtem Backen zumindest zum überwiegenden Teil aus Magnesium und/oder Titan besteht.

11. Ein Verfahren (100) nach Anspruch 10, wobei die Suspension (110) ein wasserfreies Lösungsmittel (112), vorzugsweise Alkohol, zur Einstellung der Viskosität der Suspension (110) für den Schritt des Herstellens (120) umfasst.

12. Ein Verfahren (100) nach Anspruch 10 oder 11, wobei der Schritt des Backens (130) unter Schutzgas, vorzugsweise Stickstoff oder Argon, oder im Vakuum, vorzugsweise unter 1 mbar, ausgeführt wird.

13. Ein Verfahren (100) nach einem der Ansprüche 10 bis 12, umfassend einen zusätzlichen Schritt des oberflächlichen Oxidierens (140) zur Erzeugung einer Oberflächenoxidschicht (15) auf dem Leichtmetallbauteil (1), vorzugsweise mit einer Dicke zwischen 2µm und 500µm, besonders bevorzugt von 5 - 20µm, vorzugsweise wird der Schritt des oberflächlichen Oxidierens (140) mittels Anodisation oder plasmaelektrolytische Oxidation (PEO) durchgeführt.

## Claims

1. A structured light metal component (1) made of a material preferably suitable for use as an implant, which is produced from a powder material (2) comprising magnesium and/or titanium by means of Stereolithography (SL) to form a pre-structured preform (3) and by means of a suitable baking process above a temperature suitable for breaking up an oxide layer of the powder material to form the light metal component (1), and the material of the finished structured light metal component (1) consists at least predominantly of magnesium and/or titanium,
**characterized in that**
the light metal component (1) comprises a net-like structure (11) of struts (12) made of the material of the light metal component (1) and empty spaces (13) between them, preferably with the net-like structure (11) being designed as a volume net, and wherein the net-like structure (11) comprises a plurality of crossing and/or connecting points (14) of the struts with each other.

2. The light metal component (1) according to claim 1,
**characterized in that**
the struts (12) have a diameter of 100 µm to 1500 µm and/or the empty spaces (13) have a diameter between 200 µm and 1500 µm.

3. The light metal component (1) according to one of the above claims,
**characterized in that**
**in that** the light metal component (1) comprises at least a first region (1a) and a second region (1b), with the net-like structure (11) being arranged in the first region (1a) and a compact structure (18) being arranged at least in the second region (1b).

4. The light metal component (1) according to one of the above claims,
**characterized in that**
the material of the light metal component (1) has a surface oxide layer (15).

5. The light metal component (1) according to one of the above claims,
**characterized in that**
the light metal component (1) is an implant, preferably the light metal component (1) additionally comprises a coating (16) with an antibacterial effect or an antibiotic agent (16') or comprises a proportion of up to 10% silver.

6. The light metal component (1) according to one of the above claims,
**characterized in that**
the light metal component (1) additionally comprises a coating (17) comprising a material from the group of native or artificial collagens, fibrins, natural or artificial silks, natural proteins or polymers such as PLA, PDLA, PLLA, PTFE or a sol-gel coating.

7. The light metal component (1) according to one of the previous claims,
**characterized in that**
the material consists of magnesium or a magnesium alloy comprising up to 1.5% zinc, up to 1.5% calcium, up to 5% yttrium and/or up to 4% rare earths.

8. The light metal component (1) according to one of claims 1 to 6,
**characterized in that**
the material consists of titanium or a titanium alloy, whereby the baking process is a sintering process, and the suitable temperature is between 400 degrees Celsius and the melting temperature of titanium.

9. The light metal component (1) according to one of the previous claims,
**characterized in that**
the material has a material density of more than 90%, preferably more than 97%, particularly preferably more than 99%, of the respective nominal density of magnesium or titanium.

10. A process (100) for producing a structured light metal component (1) from a material suitable for use as an implant according to claim 1 comprising the steps
- providing a suspension (110) comprising at least one organic binder (111) and a powder material (2) comprising magnesium and/or titanium in a suitable concentration and powder size, preferably with a diameter smaller than 200 µm;
- layer-by-layer production (120) of a pre-structured preform (3) from the suspension by means of stereolithography (SL) with an energy density suitable for the binder (111) to bond the powder material (2) together to form a stable pre-form; and
- baking (130) of the pre-structured preform (3) at an appropriate temperature (TB) suitable for breaking up an oxide layer of the powder material and for joining the light metal material (2) of the pre-structured preform with at least partial expulsion of the binder (111) to form the finished structured light metal component (1), which after baking consists at least predominantly of magnesium and/or titanium.

11. A process (100) according to claim 10, wherein the suspension (110) comprises an anhydrous solvent (112), preferably alcohol, for adjusting the viscosity of the suspension (110) for the step of manufacturing (120).

12. A process (100) according to claim 10 or 11, wherein the step of baking (130) is carried out under inert gas, preferably nitrogen or argon, or in vacuum, preferably below 1 mbar.

13. A process (100) according to one of the claims 10 to 12, comprising an additional step of surface oxidation (140) for producing a surface oxide layer (15) on the light metal component (1), preferably with a thickness between 2 µm and 500 µm, particularly preferably from 5 - 20 µm, preferably the step of surface oxidation (140) is carried out by means of anodization or plasma electrolytic oxidation (PEO).

## Revendications

1. Composant en métal léger (1) structuré, constitué d'un matériau de préférence adapté à une utilisation comme implant, fabriqué par stéréolithographie (SL) à partir d'un matériau pulvérulent (2), comprenant du magnésium et/ou du titane, en forme d'ébauche (3) préstructurée du composant en métal léger (1) suivant un procédé de cuisson approprié, au-delà d'une température permettant de rompre une couche d'oxyde du matériau pulvérulent, le matériau de l'élément en métal léger (1) structuré fini se composant au moins en majeure partie de magnésium et/ou de titane,
**caractérisé en ce que**
le composant en métal léger (1) est constitué d'une structure réticulaire (11) faite d'entretoises (12) dans le matériau du composant en métal léger (1) et d'espaces vides (13) entre elles, la structure réticulaire (11) formant de préférence un maillage volumique, et
ladite structure réticulaire (11) présentant plusieurs points d'intersection et/ou de liaison (14) des entretoises entre elles.

2. Le composant en métal léger (1) selon la revendication 1,
**caractérisé en ce que**
les entretoises (12) présentent un diamètre de 100 à 1 500 µm et/ou que les espaces vides (13) ont un diamètre de 200 à 1 500 µm.

3. Le composant en métal léger (1) selon l'une quelconque des revendications précédentes,
**caractérisé en ce que**
le composant en métal léger (1) comprend au moins une première zone (1a) et une seconde zone (1b), la structure réticulaire (11) étant disposée dans la première zone (1a) et une structure compacte (18) au moins dans la seconde zone (1b).

4. Le composant en métal léger (1) selon l'une quelconque des revendications précédentes,
**caractérisé en ce que**
le composant en métal léger (1) possède une couche d'oxyde superficielle (15).

5. Le composant en métal léger (1) selon l'une quelconque des revendications précédentes,
**caractérisé en ce que**
le composant en métal léger (1) est un implant et que, de préférence, le composant en métal léger (1) est pourvu d'un revêtement (16) avec activité anti-bactérienne ou d'un antibiotique (16') ou qu'il comporte un taux d'argent pouvant atteindre les 10 %.

6. Le composant en métal léger (1) selon l'une quelconque des revendications précédentes,
**caractérisé en ce que**
le composant en métal léger (1) est pourvu en outre d'un revêtement (17) étant de préférence constitué d'un matériau appartenant au groupe des collagènes natifs ou artificiels, des fibrines, des soies naturelles ou artificielles, des protéines naturelles ou des polymères tels que PLA, PDLA, PLLA, PTFE ou une couche sol-gel.

7. Le composant en métal léger (1) selon l'une quelconque des revendications précédentes,
**caractérisé en ce que**
le matériau est constitué de magnésium ou d'un alliage de magnésium, qui comprend jusqu'à 1,5 % de zinc, jusqu'à 1,5 % de calcium, jusqu'à 5 % d'yttrium et/ou jusqu'à 4 % de terres rares.

8. Le composant en métal léger (1) selon l'une des revendications 1 à 6,
**caractérisé en ce que**
le matériau est constitué de titane ou d'un alliage de titane, le procédé de cuisson étant un procédé de frittage et la température appropriée étant comprise entre 400 degrés et la température de fusion du titane.

9. Le composant en métal léger (1) selon l'une quelconque des revendications précédentes,
**caractérisé en ce que**
la densité du matériau est supérieure à 90 %, de préférence supérieure à 97 %, plus préférablement supérieure à 99 % de la densité nominale du magnésium ou du titane respectivement.

10. Procédé (100) de fabrication d'un composant en métal léger (1) structuré à partir d'un matériau approprié à une utilisation comme implant selon la revendication 1 comprenant les étapes
• de préparation d'une suspension (110) comprenant au moins un liant organique (111) et un matériau pulvérulent (2) comprenant du magnésium et/ou du titane dans une concentration et une taille de poudre appropriées, dont le diamètre est de préférence inférieur à 200 µm,
• de fabrication (120) par couches successives d'une ébauche (3) préstructurée à partir de la suspension par stéréolithographie (SL) avec une densité d'énergie adaptée qui permette au liant (111) de lier le matériau pulvérulent (2) pour former une pré-structure stable
et
• de cuisson (130) de l'ébauche (3) à une température appropriée (température selon loi de Boyle-Mariotte), permettant de rompre une couche d'oxyde du matériau pulvérulent et de lier le matériau en métal léger (2) de la pré-structure avec expulsion au moins partielle du liant (111) afin de former le composant en métal léger (1) structuré fini, qui, après cuisson, se composera au moins en majeure partie de magnésium et/ou de titane.

11. Procédé (100) selon la revendication 10, dans lequel la suspension (110) comprend un solvant anhydre (112), de préférence un alcool, pour ajuster la viscosité de la suspension (110) pour l'étape de fabrication (120).

12. Procédé (100) selon la revendication 10 ou 11, dans lequel l'étape de cuisson (130) est réalisée sous gaz de protection, de préférence azote ou argon, ou sous vide, de préférence inférieur à 1 mbar.

13. Procédé (100) selon l'une des revendications 10 à 12, comprenant une étape supplémentaire d'oxydation en surface (140) en vue de la formation d'une couche d'oxyde superficielle (15) sur le composant en métal léger (1), de préférence d'une épaisseur comprise entre 2 et 500 µm, plus préférablement entre 5 et 20 µm, l'étape d'oxydation en surface (140) étant de préférence obtenue par anodisation ou oxydation par plasma électrolytique (PEO).
